# FASCICULE DE BREVET EUROPEEN

(11) **EP 0 695 304 B1**
(45) Date de publication et mention de la délivrance du brevet: **01.10.1997**
(21) Numéro de dépôt: 94913659.2
(22) Date de dépôt: 18.04.1994
(51) Int. Cl.: C07D 493/08, G21F 9/00, C07D 323/00

(54) **CALIX[4]ARENES-COURONNES, LEUR PROCEDE DE PREPARATION ET LEUR UTILISATION POUR L'EXTRACTION SELECTIVE DU CESIUM ET DES ACTINIDES**
CALIX[4]ARENE KRONENETHER, VERFAHREN ZU IHRER HERSTELLUNG UND IHRE VERWENDUNG IN DER SELEKTIVEN EXTRAKTION VON CAESIUM UND VON ACTINIDEN
CALIX[4]ARENE CROWN ETHERS, METHOD FOR PREPARING SAME, AND USE THEREOF FOR THE SELECTIVE EXTRACTION OF CAESIUM AND ACTINIDES

(30) Priorité: 19.04.1993 FR 9304566
(43) Date de publication de la demande: 07.02.1996
(73) Titulaire: COMMISSARIAT A L'ENERGIE ATOMIQUE, 75015 Paris (FR)
(72) Inventeur: DOZOL, Jean-François, F-04660 Pierrevert (FR); ROUQUETTE, Hélène, F-04100 Manosque (FR); UNGARO, Rocco, I-43040 Vico Fertile (IT); CASNATI, Alessandro, I-43026 San Lazzaro (IT)
(74) Mandataire: Des Termes, Monique
(86) Numéro de dépôt international: FR9400432
(87) Numéro de publication internationale: WO9424138

(56) Documents cités:
- WO-A-93/12428
- US-A- 4 477 377
- JOURNAL OF THE AMERICAN CHEMICAL SOCIETY. vol. 112 , 1990 , GASTON, PA US pages 6979 - 6985 E.GHIDINI ET AL. 'COMPLEXATION OF ALKALI METAL CATIONS BY CONFORMATIONALLY RIGID, STEREOISOMERIC CALIX(4)ARENE CROWN ETHERS.'

## Description

La présente invention a pour objet des calix|4|arènes-couronnes, leur procédé de préparation et leur utilisation pour l'extraction sélective du césium et des actinides.

De façon plus précise, elle concerne des calix|4|arènes-couronnes capables d'extraire sélectivement le césium et les actinides présents à l'état de traces dans des solutions acides ayant ou non des concentrations élevées en cations, telles que les effluents aqueux provenant d'installations de retraitement de combustibles nucléaires irradiés ou les solutions de dissolution de combustibles irradiés.

Dans ces effluents, le césium 137 est l'un des produits de fission les plus nocifs du fait de sa longue période (30 ans). Il est donc intéressant de l'éliminer sélectivement des effluents liquides issus des usines de retraitement, en particulier des concentrats d'évaporateurs et des solutions acides possédant ou non une forte salinité due en particulier à la présence de nitrate de sodium.

Etant donné les propriétés chimiques très voisines du sodium et du césium, il est extrêmement difficile d'extraire sélectivement le césium présent dans ces effluents, à une concentration généralement inférieure à 10⁻⁶mol/l, alors que la concentration en sodium est d'environ 4mol/l.

Pour résoudre ce problème, on a envisagé d'extraire le césium au moyen de ligands macrocycliques tels que les para tert-butyl-calixarènes décrits dans US-A- 4 477 377. Les para tert-butyl-calixarènes utilisés sont le tétramère, l'hexamère et l'octamère, et les meilleurs résultats sont obtenus avec l'hexamère et l'octamère, le tétramère n'ayant pas une très bonne sélectivité pour séparer le césium du potassium. Cette technique d'extraction du césium est intéressante mais elle a pour principal inconvénient de ne s'appliquer qu'au traitement de solutions aqueuses basiques, alors que la plupart des effluents provenant du retraitement sont des solutions acides.

D'autres ligands macrocycliques tels que les éthers-couronnes ont également été utilisés dans ce but comme il est décrit dans Selective Extraction of Cesium from Acidic Nitrate Solutions with Didodecylnaphtalene sulphonic acid synergized with bis(tert-butylbenzo)-21 crown 7. W. J. Mc Dowell and al, Anal. Chem. 1992, 64, 3013-3017, mais leur sélectivité vis-à-vis du césium reste faible.

Des eaux[4jarènes-éther couronnes utilisables comme colorants sensibles aux ions sont décrits dans le document WO-A.-93/12428 publié le 24 juin 1993. Ces calixarènes peuvent être préparés à partir de calix[4jarènes-éther couronnes de formule : 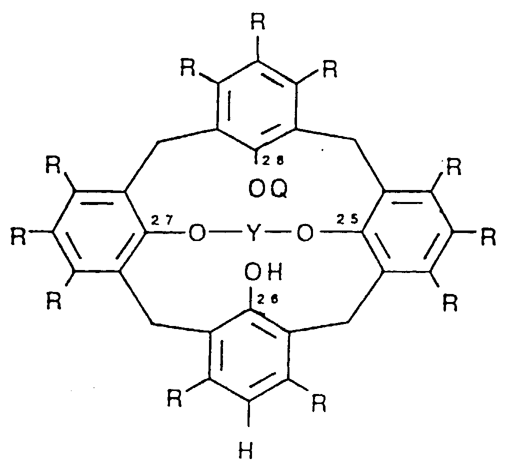 dans laquelle les R sont indépendamment l'hydrogène ou un groupe alkyle, Q est l'hydrogène ou un groupe alkyle, et Y représente -(CH₂)₂[Q(CH₂)₂]n- avec n étant un entier de 1 à 6.

La présente invention a précisément pour objet de nouveaux calixarènes qui permettent d'extraire sélectivement le césium à partir de solutions acides et de le séparer du sodium avec une bonne efficacité.

Selon l'invention, les calixarènes sont des calix|4|arènes-couronnes de formule : 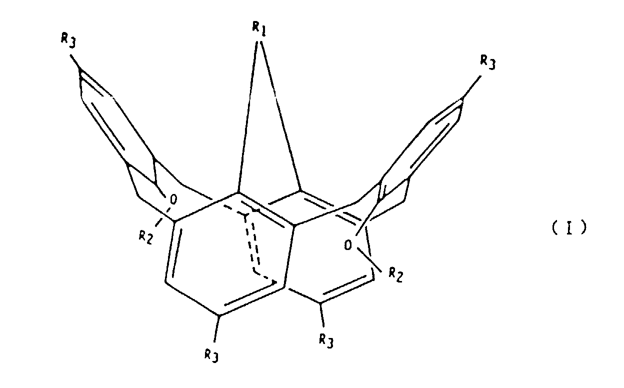 dans laquelle
- R₁ représente un groupe de formule X(CH₂CH₂X)ₘ ou de formule X(CH₂CH₂X)ₙYX(CH₂CH₂X)ₙ, dans lesquelles X représente O et/ou N(R₄) avec R₄ étant un atome d'hydrogène ou un groupe alkyle en C₁ à C₄, Y représente un groupe cycloalkylène en C₆ à C₁₄ ou arylène comportant éventuellement un hétéroatome, choisi parmi O, S et N, m est égal à 3, 4, 5 ou 6 et n est égal à 1, 2 ou 3.
- R₂ représente un atome d'hydrogène, un groupe alkyle en C₁ à C₁₂, un groupe de formule 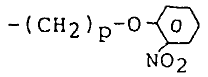 avec p étant égal à 5, 6, 7 ou 8, -CH₂-CH₂OH ou -CH₂COR₅ avec R₅ représentant un groupe de formule OR₆, NR₆R₇ ou R₆ dans lesquelles R₆ et R₇ qui peuvent être identiques ou différents, représentent un atome d'hydrogène, un groupe alkyle en C₁ à C₄ ou un groupe aryle en C₆ à C₁₄, les deux R₂ pouvant être différents, et
- R₃ représente un atome d'hydrogène ou un groupe alkyle en C₁ à C₃,
à condition que l'un ou les deux R₂ ne représentent pas un atome d'hydrogène lorsque R₁ représente X(CH₂CH₂X)m avec X = 0.

Dans la formule donnée, ci-dessus, les groupes alkyle utilisés pour R₂, R₄, R₆ et R₇ peuvent être linéaires ou ramifiés et ne sont pas nécessairement identiques, en particulier pour R₂.

Les groupes alkyle utilisés pour R₄, R₆ et R₇ sont des groupes alkyle ayant de 1 à 4 atomes de carbone.

En revanche, les groupes alkyle utilisés pour R₂ peuvent comporter davantage d'atomes de carbone, à savoir de 1 à 12 atomes de carbone, de préférence de 1 à 8 atomes de carbone.

Les groupes aryle utilisés pour R₆ et R₇ ont de 6 à 14 atomes de carbone. A titre d'exemples de tels groupes, on peut citer les groupes phényle et naphtyle.

Dans la formule donnée ci-dessus, on entend par groupe "cycloalkylène", un groupe bivalent dérivé d'un hydrocarbure cyclique par enlèvement d'un atome d'hydrogène à chacun de deux atomes de carbone du cycle. A titre d'exemple d'un tel groupe, on peut citer le groupe cyclohexylène. Les groupes cycloalkylène utilisés pour Y comportent de 6 à 14 atomes de carbone.

De même, on entend par groupe "arylène", un groupe dérivé d'un hydrocarbure aromatique comportant un ou plusieurs noyaux aromatiques ou d'un noyau hétérocyclique comportant un hétéroatome choisi parmi O, S ou N, par enlèvement d'un atome d'hydrogène à chacun de deux atomes de carbone du cycle.

A titre d'exemple de tels groupes, on peut citer les groupes phénylène, naphtylène, benzylène, pyridylène et thiénylène.

Dans le chaînon R¹ les X peuvent représenter des atomes d'oxygène qui peuvent être remplacés partiellement ou totalement par des atomes d'azote NR⁴.

Les calixarènes de l'invention sont très différents des calixarènes utilisés dans US-A-4 477 377. En effet, dans ce document, on utilisait des calixarènes comportant sur chaque noyau benzénique un groupement hydroxyle et un groupement tert-butyle, et ces calixarènes ne comportaient qu'un seul macrocycle.

En revanche, dans les calixarènes de l'invention, il y a deux cycles, constitués respectivement par un premier cycle correspondant à l'ensemble des noyaux benzéniques reliés entre eux par les groupes CH₂, et un second cycle formé par un pont éther-couronne ou aza-couronne entre deux noyaux benzéniques diamétralement opposés.

De préférence, le second cycle est formé par un pont éther-couronne de formule O(CH₂CH₂O)ₘ avec m=5 ou 6.

Grâce à leur structure particulière, les calixarènes de l'invention allient les propriétés complexantes des éthers-couronnes vis-à-vis des alcalins avec les propriétés des calixarènes, c'est-à-dire une importante sélectivité imposée par la structure rigide de la cavité et un caractère lipophile élevé qui rendent la molécule particulièrement intéressante pour l'utilisation dans une membrane liquide supportée.

Dans les calixarènes de l'invention, R₃ représente avantageusement un atome d'hydrogène.

Lorsque R₂ n'est pas un groupe alkyle, il peut être par exemple le groupe -CH₂CH₂OH ou le groupe -CH₂COOCH₂CH₃.

Les calixarènes de formule I avec R₂ représentant un atome d'hydrogène peuvent être préparés par un procédé consistant à faire réagir un calix|4|arène de formule : 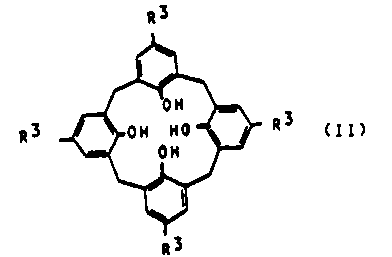 dans laquelle R₃ a la signification donnée ci-dessus,
- avec un composé répondant à l'une des formules :

   TsX(CH₂CH₂X)ₘTs (III)

   et

   TsX(CH₂CH₂X)ₙYX(CH₂CH₂X)ₙTs (IV)

   dans lesquelles X, m, Y et n ont les significations données ci-dessus, et Ts représente CH₃-C₆H₄-SO₂ et à séparer du milieu réactionnel le calix|4|arène-couronne obtenu.

Les calix|4|arènes-couronnes répondant à la formule (I) donnée ci-dessus avec R₂ différent d'un atome d'hydrogène peuvent être préparés par un procédé comprenant deux étapes à partir du calix|4|arène de formule (II) en réalisant une étape de substitution de celui-ci par des groupes R₂ et une étape de formation du pont éther-couronne ou aza-couronne R₁.

Selon un premier mode de réalisation de ce procédé, celui-ci comprend les étapes suivantes :
a) faire réagir un calix|4|arène de formule : 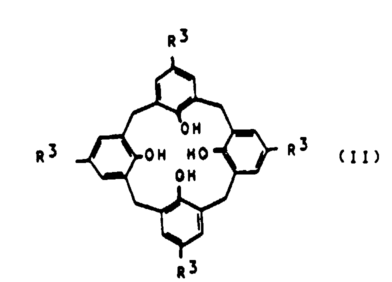 dans laquelle R₃ a la signification donnée ci-dessus, avec un halogénure de formule R₂Z
   dans laquelle R₂ a la signification donnée ci-dessus et Z est un atome d'halogène,
b) faire réagir le calix|4|arène obtenu dans l'étape a) avec un composé répondant à l'une des formules suivantes :

   TsX(CH₂CH₂X)ₘTs (III)

   et

   TsX(CH₂CH₂X)ₙYX(CH₂CH₂X)ₙTs (IV)

   dans lesquelles X, m, Y, n et Ts ont les significations données ci-dessus, et
c) séparer du milieu réactionnel le calix|4|arène-couronne obtenu.

Ce procédé, dénommé procédé A, est illustré schématiquement ci-après.

Avec ce procédé, on part du composé 1 de formule (II), on le substitue partiellement par R₂ pour obtenir le composé 2 et l'on forme ensuite le pont éther-couronne ou aza-couronne pour obtenir le composé 4, c'est-à-dire le calix|4|arène-couronne de formule (I).

Selon un second mode de réalisation, le procédé comprend les étapes suivantes :
a) faire réagir un calix|4|arène de formule : 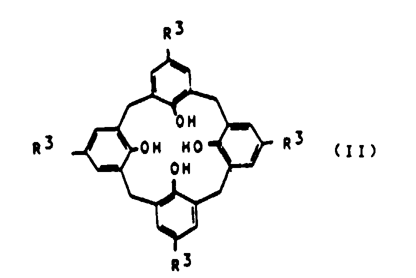 dans laquelle R₃ a la signification donnée ci-dessus,
   - avec un composé répondant à l'une des formules suivantes :

      TsX(CH₂CH₂X)ₘTs (III)

      et

      TsX(CH₂CH₂X)ₙYX(CH₂CH₂X)ₙTs (IV)

      dans lesquelles X, m, Y, Ts et n ont les significations données ci-dessus,
b) faire réagir le calix|4|arène-couronne obtenu dans l'étape a) avec un halogénure de formule R₂Z dans laquelle R₂ a la signification donnée ci-dessus et Z est un atome d'halogène, et
c) séparer du milieu réactionnel le calix|4|arène-couronne de formule (I) ainsi obtenu.

Ce procédé, dénommé procédé B est illustré également ci-après. Dans ce cas, on part également du composé 1, mais on commence par former le pont éther-couronne ou aza-couronne R₁ pour obtenir le composé 3 que l'on substitue ensuite par R₂ pour obtenir le composé 4.

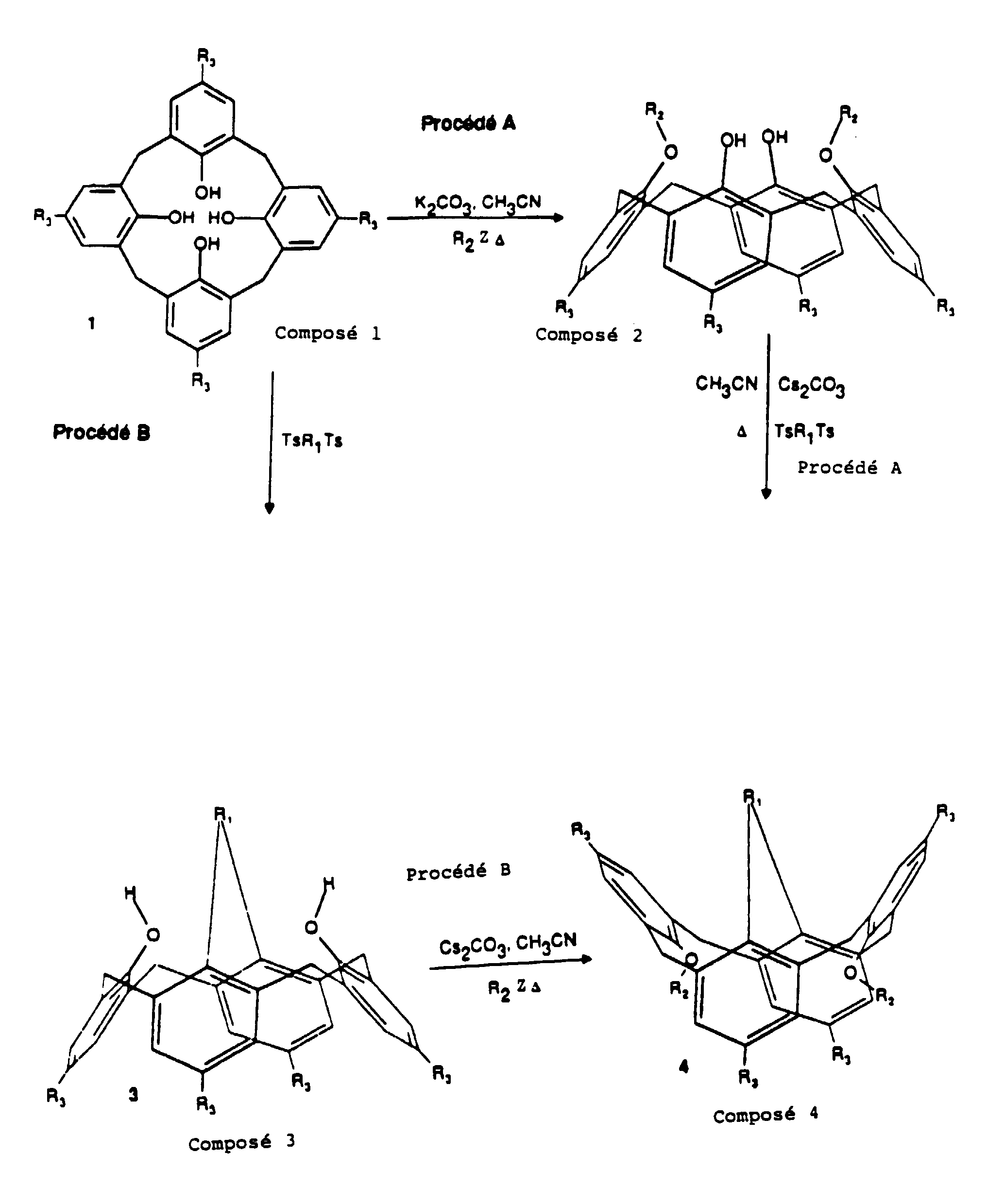

Pour effectuer la réaction de formation du pont R₁, on dissout le calixarène dans un solvant approprié, par exemple dans le benzène ou l'acétonitrile, on y ajoute un seL tel que le carbonate de potassium et le diparatoluènesulfonate de formule (III) ou (IV) et on laisse réagir au reflux pendant une durée suffisante pour former le pont R₁ reliant deux noyaux benzéniques opposés. Après réaction, on dissout le mélange réactionnel dans un solvant approprié et on extrait le calixarène, par exemple au moyen d'acide chlorhydrique.

Pour effectuer la réaction de substitution du calixarène par R₂, on peut opérer également dans un solvant tel que l'acétonitrile en ajoutant du carbonate de potassium et l'halogénure R₂Z, par exemple l'iodure.

Les calix|4|arènes couronnes de formule (I) décrits précédemment peuvent être utilisés en particulier pour l'extraction sélective du césium présent dans des solutions aqueuses, notamment des solutions acides chargées ou non en sodium et/ou strontium telles que les solutions de dissolution et les effluents aqueux provenant d'installations de retraitement de combustibles irradiés.

Lorsque le calix|4|-arène-couronne utilisé comporte des substituants alkyle sur les noyaux benzéniques, il peut être préparé par un procédé analogue à celui décrit ci-dessus en partant du calix|4|arène substitué correspondant.

Un calix|4|arène-couronne substitué de ce type avec R³ représentant le groupe tert-butyle est décrit par exemple par Ghidini et al dans J. Am. Chem. Soc., 1990, 112, p. 6979-6985.

Pour l'extraction du césium, les solutions aqueuses de départ peuvent être des solutions acides, par exemple des solutions nitriques contenant de 10⁻³ à 7mol/l d'acide nitrique.

Pour réaliser cette extraction, on met en contact la solution aqueuse contenant le césium avec une phase liquide immiscible comprenant le calix|4|arène-couronne, puis on récupère le césium extrait dans la phase liquide immiscible.

Cette récupération peut être effectuée au moyen d'une solution aqueuse par mise en contact de la phase liquide immiscible ayant extrait le césium avec une solution aqueuse de réextraction, constituée par exemple par de l'eau distillée et désionisée.

Pour mettre en oeuvre ce procédé, on peut effectuer la mise en contact de la solution aqueuse avec la phase liquide immiscible dans des installations classiques d'extraction liquide-liquide telles que des mélangeurs-décanteurs, des colonnes pulsées, etc.

On peut aussi effectuer cette mise en contact en disposant la phase liquide immiscible comprenant le calix|4|arène-couronne sous la forme d'une membrane liquide comportant deux surfaces opposées, la solution aqueuse de départ contenant le césium étant au contact d'une des surfaces de la membrane, en recueillant le césium extrait par la membrane liquide dans une solution aqueuse de réextraction au contact de la surface opposée de la membrane.

Pour former la phase liquide immiscible, on dissout le calix|4|arène couronne dans un solvant approprié.

A titre d'exemple de solvants utilisables, on peut citer les alkylbenzènes et les nitrophényl alkyl éthers.

De préférence, on utilise comme solvant un éther tel que l'ortho-nitrophénylhexyléther et l'ortho-nitrophényloctyléther.

La concentration en calix|4|arène couronne de la phase liquide immiscible, dépend en particulier du solvant utilisé. On peut utiliser des concentrations allant de 10⁻³ à 5.10⁻¹mol/l, par exemple une concentration de 10⁻²mol/l.

Les calix|4|arène-couronne de formule (I) peuvent également être utilisés pour séparer certains actinides de l'américium trivalent. Dans ce cas, on met également en contact la solution aqueuse contenant les actinides avec une phase liquide immiscible contenant le calix|4|arène-couronne pour extraire sélectivement les actinides en conservant l'américium dans la solution aqueuse.

On précise que pour l'extraction du césium comme pour celle des actinides, on peut utiliser les calixarènes sous la forme d'isomères purs ou de mélanges d'isomères. On peut aussi utiliser des mélanges de calixarènes.

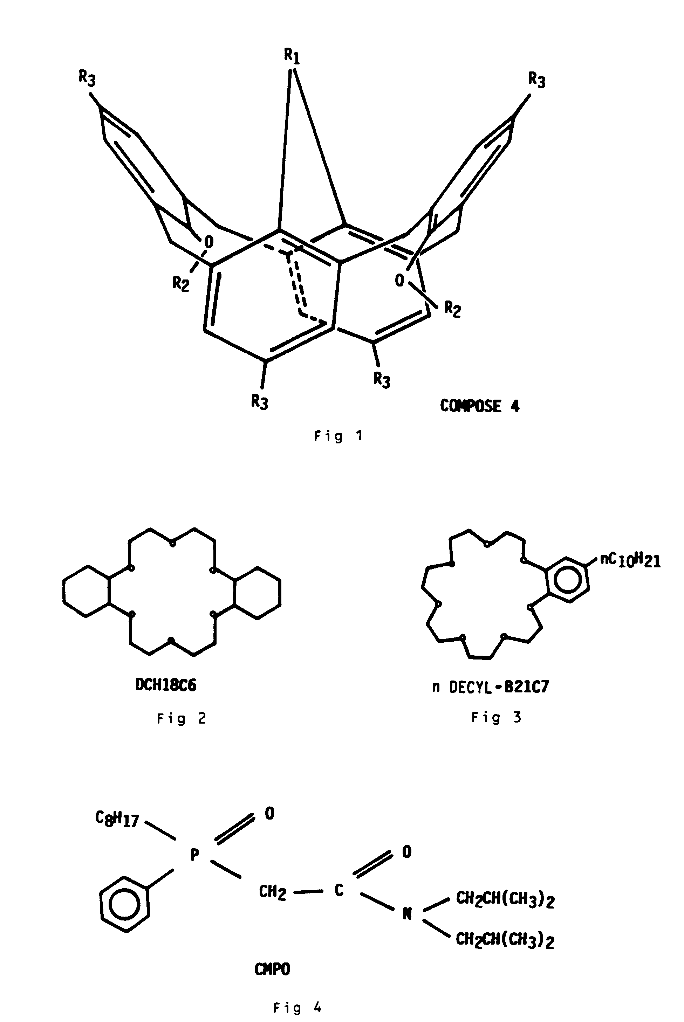

D'autres caractéristiques et avantages de l'invention apparaîtront mieux à la lecture des exemples suivants donnés bien entendu à titre illustratif et non limitatif en référence au dessin annexé qui représente les différents ligands utilisés dans ces exemples.

### Exemple 1 : Préparation du 25, 27-dihydroxycalix-|4|arène-couronne-6 (Composé 4 avec R₁ = O(CH₂CH₂O)₅, R₂ = H et R₃ = H).

On dissout dans 100ml de benzène sec, 1,0g (2,35mmol) de calix|4|arène (composé 1 avec R₃=H), on le porte au reflux sous atmosphère d'azote avec 0,53g (4,7mmol) de tert-butoxyde de potassium, et on y ajoute goutte à goutte sur une période de 6h une solution contenant 1,28g (2,35mmol) de di-p-toluène sulfonate de pentaéthylène glycol dans 50ml de benzène sec.

Après 36h au reflux, on laisse le mélange réactionnel refroidir, on le traite avec HCl à 10% et on lave deux fois à l'eau la couche organique séparée. Finalement, on élimine le solvant sous pression réduite et on chromatogrpahie le résidu sur une colonne de gel de silice en utilisant comme éluant un mélange hexane/acétate d'éthyle (1/1). On obtient ainsi le 25,27-dihydroxycalix|4|arène-couronne-6 avec un rendement de 34%.

Les caractéristiques du produit sont les suivantes :
- point de fusion : 224-225°C.
- RMN du proton dans CDCl₃ : δ 7,49 (s, 2H, OH), 7,07 (d, 4H, ArH meta, J = 7,6Hz), 6,83 (t, 4H, ArH meta, J = 7,6Hz), 6,70 (t, 2H, ArH, ArH para, J = 7,6Hz), 6,68 (t, 2H, ArH para, J = 7,6Hz), 4,42 (d, 4H, ArCH₂Ar, J = 13,1Hz), 4,15 (t, 4H, ArOCH₂CH₂O(CH₂CH₂O)₃CH₂CH₂OAr, J = 4,8Hz), 4,01 (t, 4H, ArOCH₂CH₂O(CH₂CH₂O)₃CH₂CH₂OAr, J = 4,8Hz), 3,93 (t, 4H, ArOCH₂CH₂OCH₂CH₂OCH₂CH₂OCH₂CH₂OCH₂CH₂OAr, J = 4,8Hz), 3,84 (t, 4H, ArOCH₂CH₂OCH₂CH₂OCH₂CH₂OCH₂CH₂OCH₂CH₂OAr, J = 4,8Hz), 3,70 (s, 4H, ArO(CH₂CH₂O)₂CH₂CH₂O(CH₂CH₂O)2Ar), 3,36 (d, 4H, ArCH₂Ar, J = 13,1Hz).
- Spectrométrie de masse (CI) 626,0 (M⁺ calculée 626,3)

| - Analyse élémentaire | | |
|---|---|---|
| pour C₃₈H₄₂O₈ | C | H |
| calculé | 72,83 | 6,75 |
| trouvé | 72,71 | 6,91 |

### Exemple 2 : Préparation du 25,27 -diméthoxycalix|4|arène-couronne-6 (composé 3 avec R₁ = O(CH₂CH₂O)₅, R₂ = CH₃, R₃ = H).

### Procédé A

A une solution contenant 1,0g (2,35mmol) de calix|4|arène (composé 1 avec R₃ = H) dans 100ml d'acétonitrile sec, on ajoute sous atmosphère d'azote 0,35g (2,5mmol) de carbonate de potassium et 0,67g (4,7mmol) d'iodométhane. On porte le mélange réactionnel au reflux pendant 24h, puis on élimine l'acétonitrile sous pression réduite et on trempe le résidu dans 100ml de HCl à 10% et 100ml de dichlorométhane. On sépare la couche organique, on la lave deux fois avec de l'eau distillée, puis on élimine le dichlorométhane par distillation. On ajoute alors du méthanol et on filtre le précipité. On obtient ainsi 1,03g (2,28mmol) d'un solide blanc qui est le 25,27-diméthoxycalix|4|arène, c'est-à-dire le composé 2 avec R₂ = CH₃ et R₃ = H. On sèche ce produit solide sous vide poussé pendant quelques heures, puis on le dissout dans 400ml d'acétonitrile, et on ajoute 2,93g (9mmol) d'un excès de carbonate de césium et 1,37g (2,5mmol) de di-p-toluène sulfonate de pentaéthylène glycol sous atmosphère d'azote. On porte le mélange réactionnel au reflux pendant 16h, puis on le trempe et on le traite comme ci-dessus. Par cristallisation du résidu huileux dans un mélange de dichlorométhane/méthanol (1/5), on obtient le 25,27-diméthoxycalix|4|arène-couronne-6 (composé 4 avec R₁ = O(CH₂CH₂O)₅, R₂ = CH₃ et R₃ = H) avec un rendement total de 78%.

Les caractéristiques de ce produit sont les suivantes :
- point de fusion : 180 - 181°C.
- RMN du proton (CDCl₃) : δ 7,15 (d, 4H, ArH meta, J = 6,5Hz), 6,93 (t, 2H, ArH para, J = 6,5HZ), 6,93 (s, 6H, ArH), 4,43 (d, 4H, ArCH₂Ar, J = 12,9Hz), 4,05 (s, 4H, O(CH₂CH₂O)₂CH₂CH₂(OCH₂CH₂)₂O), 3,89 (s, 6H, OCH₃), 3,8-3,5 (m, 16H, O(CH₂CH₂O)2CH2CH2(OCH₂- CH₂)₂O), 3,15 (d, 4H, ArCH₂Ar, J = 12,9Hz). - Spectrométrie de masse (CI) 654,0 (M⁺ calculée 654,3).

| - Analyse élémentaire pour | | |
|---|---|---|
| C₄₀H₄₆O₈ | | |
| calculé | 73,37 | 7,08 |
| trouvé | 73,80 | 7,20 |

### Procédé B

On dissout 0,51g (0,81mmol) du 25,27-dihydroxycalix|4|arène-couronne-6 préparé dans l'exemple 1 dans 200ml d'acétonitrile sec. On ajoute à cette solution sous agitation lg (3,2mmol) de carbonate de césium et 0,69g (4,8mmol) d'iodométhane. Après 24h, on élimine l'acétonitrile sous pression réduite et on traite le résidu avec 70ml de dichlorométhane et 70ml de HCl à 10%. On sépare la phase organique, on la lave deux fois avec de l'eau et on élimine le solvant par distillation. Après cristallisation du résidu dans un mélange de dichlorométhane/méthanol (1/5), on obtient le 25,27-diméthoxycalix|4|arène-couronne-6 pur (composé 4 avec R₁ = O(CH₂CH₂O)₅, R₂ = CH₃, R₃ = H) avec un rendement total de 33%.

Les caractéristiques du produit obtenu sont les mêmes que celles du produit obtenu par le procédé A.

### Exemple 3 : Préparation du 25,27-bis(1-propyloxy)calix-|4|arène-couronne-6 (composé 4 avec R₁ = O(CH₂CH₂O)₅, R₂ = CH₂CH₂CH₃ et R₃ = H).

### Procédé A.

A une solution de 1,0g (2,35mmol) de calix|4|arène (composé 1 avec R₃ = H) dans 100ml d'acétonitrile sec, on ajoute 1,30g (9,4mmol) de carbonate de potassium et 1,60g (9,4mmol) de 1-iodopropane sous atmosphère d'azote. On porte le mélange réactionnel au reflux pendant 36h, puis on élimine l'acétonitrile sous pression réduite et on trempe le résidu dans 100ml de HCl à 10% et 100ml de dichlorométhane. On sépare la couche organique, on la lave deux fois avec de l'eau distillée et on élimine ensuite le dichlorométhane par distillation. On ajoute alors du méthanol et on filtre le précipité. On obtient ainsi 1,02g (2,0mmol) d'un produit solide blanc qui est le 25,27-bis-(1-propyloxy)calix|4|arène (composé 2 avec R₂ = CH₂CH₂CH₃ et R₃ = H). On sèche alors le solide sous vide poussé pendant plusieurs heures, on le dissout dans 400ml d'acétonitrile et on ajoute 2,6g (8,0mmol) d'un excès de carbonate de césium et 1,20g (2,2mmol) de di-p-toluènesulfonate de pentaéthylène glycol sous atmosphère d'azote. On porte le mélange réactionnel au reflux pendant 16h, puis on le trempe et on le traite comme ci-dessus. Par cristallisation du résidu huileux obtenu dans du méthanol, on obtient le 25,27-bis(l-propyloxy)calix|4|arène-couronne-6 pur (composé 4 avec R₁ = O(CH₂CH₂O)₅, R₂ = CH₂CH₂CH₃ et R₃ = H) avec un rendement total de 64%.

Les caractéristiques de ce produit sont les suivantes :
- point de fusion : 140-141°C.
- RMN du proton (CDCl₃) : δ 7,15-6,95 (m, 8H, ArH meta), 6,9-6,6 (m, 4H, ArH para), 3,77-3,33 (m, 32H, O(CH₂CH₂O)₅, OCH₂CH₂CH₃, ArCH₂Ar), 1,28 (sext, 4H, OCH₂CH₂CH₃, J = 7,7HZ), 0,73 (t, 6H, OCH₂CH₂CH₃).
- Spectrométrie de masse (CI) 710,2 (M⁺ calculée 710,4).

| - Analyse élémentaire pour | | |
|---|---|---|
| C₄₄H₅₄O₈ | C | H |
| calculé | 74,33 | 7,65 |
| trouvé | 74,25 | 7,83 |

### Procédé B

On dissout 0,51g (0,81mmol) du 25,27-dihydroxycalix|4|arène-couronne-6 (composé 3) préparé dans l'exemple 1 dans 200ml d'acétonitrile sec. On ajoute à cette solution sous agitation 1,06g (3,2mmol) de carbonate de césium et 0,82g (4,8mmol) de 1-iodopropane. Après 24h, on élimine l'acétonitrile sous pression réduite et on traite le résidu avec 70ml de dichlorométhane et 70ml de HCl à 10%. On sépare la phase organique, on la lave deux fois avec de l'eau et on élimine le solvant par distillation. Par cristallisation dans du méthanol, on obtient le 25,27-bis(l-propyloxy)calix|4|arène-couronne-6 pur (composé 4 avec R₁ = O(CH₂CH₂O)₅, R₂ = CH₂CH₂CH₃ et R₃ = H) avec un rendement total de 31%.

Les caractéristiques de ce produit sont identiques à celles du produit obtenu par le procédé A.

### Exemple 4 : Préparation du 25,27-bis(2-propyloxy)calix-|4|arène-couronne-6 (composé 4 avec R₁ = O(CH₂CH₂O)₅, R₂ = CH(CH₃)₂ et R₃ = H).

### Procédé A.

A une solution de 1,0g (2,35mmol) de calix|4|arène (composé 1 avec R₃ = H) dans 100ml d'acétonitrile sec, on ajoute sous atmosphère d'azote 0,35g (2,5mmol) de carbonate de potassium et 1,60g (9,4mmol) de 2-iodopropane. On porte le mélange réactionnel au reflux pendant 48h, puis on élimine l'acétonitrile sous pression réduite et on trempe le résidu dans 100ml de HCl à 10% et 100ml de dichlorométhane. On sépare la couche organique, on la lave deux fois avec de l'eau distillée, puis on élimine le dichlorométhane par distillation. On ajoute alors du méthanol et on filtre le précipité. Le solide blanc résultant (0,97g, 1,9mmol) correspondant au 25,27-bis(2-propyloxy)calix|4|arène (composé 2 avec R₂ = CH(CH₃)₂ et R₃ = H) est séché sous vide poussé pendant plusieurs heures, puis dissous dans 400ml d'acétonitrile. On ajoute alors 2,45g (7,52mmol) d'un excès de carbonate de césium et 1,04g (1,90mmol) de di-p-toluène sulfonate de pentaéthylène glycol sous atmosphère d'azote. On porte le mélange réactionnel au reflux pendant 16h, puis on le trempe et on le traite comme ci-dessus. On obtient par cristallisation du résidu huileux dans l'éthanol du 25,27-(bis-2-propyloxy)calix|4|arène-couronne-6 pur (composé 4 avec R₁ = O(CH₂CH₂O)₅, R₂ = CH(CH₃)₂ et R₃ = H) avec un rendement de 60%.

Les caractéristiques de ce produit sont les suivantes :
- point de fusion : 197-198°C.
- RMN du proton (CDCl₃) : δ 7,1-7,0 (m, 8H, ArH meta), 6,9-6,7 (m, 4H, ArH para), 4,23 (ept, 2H, OCH(CH₃)₂, J = 6,3Hz), 3,8-3,3 (m, 28H, O(CH₂CH₂O)₅, ArCH₂Ar), 0,90 (d, 12H, OCH(CH₃)₂, J = 6,3Hz).
- Spectrométrie de masse (CI) 710,1 (M⁺ calculée 710,4)

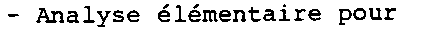

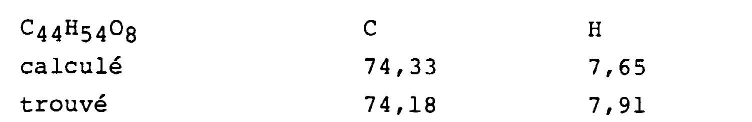

### exemple 5 : Préparation du 25,27-diméthoxycalix|4|arène-couronne 7 (composé 4 avec R₁ = O(CH₂CH₂O)₆, R₂ = CH₃, R₃ = H).

### Procédé A.

A une solution de 1,0 g (2,35mmol) de calix-|4|arène (composé 1 avec R₃ = H), dans 100ml d'acétonitrile sec, on ajoute sous atmosphère d'azote 0,36g (2,5mmol) de carbonate de potassium et 0,67g (4,7mmol) d'iodométhane. On porte le mélange réactionnel au reflux pendant 24h, puis on élimine l'acétonitrile sous pression réduite et on trempe le résidu dans 100ml de HCl à 10% et 100ml de dichlorométhane. On sépare la couche organique, on la lave deux fois avec de l'eau distillée et on élimine le dichlorométhane par distillation. On ajoute alors du méthanol et on filtre le précipité. Le solide blanc résultant (1,03g, 2,28mmol) correspondant au 25,27-diméthoxycalix|4|arène (composé 2 avec R₂ = CH₃ et R₃ = H) est séché sous vide poussé pendant plusieurs heures, puis est dissous dans 400ml d'acétonitrile. On ajoute alors sous atmosphère d'azote 2,97g (9mmol) d'un excès de carbonate de césium et 1,48g (2,5mmol) de di-p-toluène sulfonate d'hexaéthylène glycol. On porte le mélange réactionnel au reflux pendant 16h, on le trempe et on le traite comme ci-dessus. Par cristallisation du résidu huileux dans un mélange de dichlorométhane/méthanol (1/5), on obtient le 25,27-diméthoxycalix|4|arène-couronne-7 pur (composé 4 avec R₁ = O(CH₂CH₂O)₆, R₂ = CH₃ et R₃ = H) avec un rendement total de 75%.

Les caractéristiques du produit sont les suivantes :
- Point de fusion : 121-122°C.
- RMN du proton (CDCl₃) : δ 7,4-6,7 (m, 8H, ArH), 6,6-6,35 (m, 4H, ArH), 4,50 (d, 4H, ArCH₂Ar, J = 13,0Hz), 4,02-3,10 (m, 34H, O(CH₂CH₂O)₆, OCH₃, ArCH₂Ar.
- Spectrométrie de masse (CI) 698,0 ((M⁺ calculée 698,3).

| - Analyse élémentaire pour | | |
|---|---|---|
| C₄₂H₅₀O₉ | C | H |
| calculé | 72,18 | 7,21 |
| trouvé | 71,96 | 7,43 |

### Exemple 6 : Préparation du 25,27-bis(1-octyloxy)calix|4|arène-couronne-6 (composé 4 avec R₁ = O(CH₂CH₂O)₅, R₂ = (CH₂)₇CH₃ et R₃ = H).

### Procédé A.

A une solution de 1,0g (2,35mmol) de calix|4|arène (composé 1 avec R₃ = H), dans 100ml d'acétonitrile sec, on ajoute sous atmosphère d'azote 0,75g (5,4mmol) de carbonate de potassium et 0,99g (5,17mmol) de 1-bromooctane. On porte le mélange réactionnel au reflux pendant 6 jours, puis on élimine l'acétonitrile sous pression réduite et on trempe le résidu dans 100ml de HC1 à 10% et 100ml de dichlorométhane. On sépare la couche organique, on la lave deux fois avec de l'eau distillée, on élimine le dichlorométhane par distillation et on traite le résidu avec de l'éther de pétrole froid. Le solide blanc résultant (0,70g, 1,08mmol) correspondant au 25,27-bis(l-octyloxy)calix|4|arène (composé 2 avec R₂ = (CH₂)₇CH₃ et R₃ = H) est séché sous vide poussé pendant plusieurs heures, puis dissous dans 400ml d'acétonitrile. On ajoute alors 1,40g (4,3mmol) d'un excès de carbonate de césium et 0,65g (1,19mmol) de di-p-toluène sulfonate de pentaéthylène glycol sous atmosphère d'azote. On porte le mélange réactionnel au reflux pendant 16h et on le trempe et on le traite comme ci-dessus. Par chromatographie sur colonne de gel de silice du résidu huileux obtenu, en utilisant comme éluant un mélange d'hexane et d'acétate d'éthyle (1/1) et cristallisation dans du méthanol, on obtient le 25,27-bis(l-octyloxy)calix|4|arène-couronne-6 pur (composé 4 avec R₁ = O(CH₂CH₂O)₅, R₂ = (CH₂)₇CH₃ et R₃ = H) avec un rendement total de 35%.

Les caractéristiques de ce produit sont les suivantes :
- Point de fusion : 94-95°C.
- RMN du proton (CDCl₃) : δ 7,12 (d, 4H, ArH meta, J = 7,5Hz), 7,08 (d, 4H, ArH meta, J = 7,5Hz), 6,83 (t, 2H, ArH para, J = 7,5Hz), 6,77 (t, 2H, ArH para, J = 7,5Hz), 3,78 (s, 8H, ArCH₂Ar), 3,71 (s, 4H, O(CH₂CH₂O)₂CH₂CH₂(OCH₂CH₂)₂O), 3,66, 3,60, 3,49 et 3,40 (t, chaque 4H, O(CH₂CH₂O)₂CH₂CH₂(OCH₂CH₂)₂O), 3,43 (t, 4H, OCH₂(CH₂)₆CH₃, J = 7,4Hz), 1,36-1,15 (m, 24H, OCH₂(CH₂)₆CH₃), 0,92 (t, OCH₂(CH₂)₆CH₃, J = 7,1Hz).
- Spectrométrie de masse (CI) 850,2 (M⁺ calculée 850,5).

| - Analyse élémentaire pour | | |
|---|---|---|
| C₅₄H₇₄O₈ | C | H |
| calculé | 76,20 | 8,76 |
| trouvé | 76,02 | 8,86 |

### Exemple 7 : Préparation du 25,27-bis(éthoxycarbonylméthoxy)calix|4|arène-couronne-6 (composé 4 avec R₁ = O(CH₂CH₂O)₅, R₂ = CH₂COOCH₂CH₃ et R₃ = H).

### Procédé A.

A une solution de 1,0g (2,35mmol) de calix|4|arène (composé 1 avec R₃ = H) dans 100ml d'acétonitrile sec, on ajoute sous atmosphère d'azote 0,36g (2,6mmol) de carbonate de potassium et 0,82g (4,94mmol) de monobromoacétate d'éthyle. On porte le mélange réactionnel au reflux pendant 24h, puis on élimine l'acétonitrile sous pression réduite et on trempe le résidu dans 100ml de HCl à 10% et 100ml de dichlorométhane. On sépare la couche organique, on la lave deux fois à l'eau distillée et on élimine le dichlorométhane par distillation. On traite le résidu avec du méthanol et on filtre le précipité. Le solide blanc résultant (1,26g, 2,12mmol) correspondant au 25,27-bis(éthoxycarbonylméthoxy)calix|4|arène (composé 2 avec R₂ = CH₂COOCH₂CH₃), R₃ = H) est séché sous vide poussé pendant plusieurs heures puis dissous dans 400ml d'acétonitrile. On ajoute sous atmosphère d'azote 2,76g (8,46mmol) d'un excès de carbonate de césium et 1,28g (2,33mmol) de di-p-toluènesulfonate de pentaéthylène glycol. On porte le mélange réactionnel au reflux pendant 16h, puis on le trempe et on le traite comme ci-dessus. Par chromatographie sur colonne de gel de silice du résidu huileux, en utilisant comme éluant l'acétate d'éthyle, on obtient le 25,27-bis(éthoxycarbonylméthoxy)calix|4|arène-couronne-6 pur (composé 4 avec R₁ = O(CH₂CH₂O)₅, R₂ = CH₂COOCH₂CH₃ et R₃ = H) avec un rendement total de 45% sous la forme d'un produit vitreux.

Les caractéristiques de ce produit sont les suivantes :
- RMN du proton (CDCl₃) δ 7,12 (d, 8H, ArH meta, J = 7,2Hz), 6,89 (t, 2H, ArH para), 6,80 (t, 2H, ArH para), 4,3-3,3 (m, 36H, ArCH₂Ar, O(CH₂CH₂O)₅, OCH₂COOCH₂CH₃), 1,20 (t, 6H, OCH₂CCH₃, J = 7,6Hz) - ¹³C RMN (CDCl₃) : δ 170,1 (s, C=O), 157,0 et 155,3 (s, ArO), 134,6 et 133,8 (s, Ar ortho), 130,4 (d, Ar meta), 123,1 et 122,8 (d, Ar para), 71,1, 70,9, 70,6, 70,0, 69,3, 68,2 (t, OCH₂CH₂O)₅ et OCH₂CO), 60,22 (t, OCH₂CH₃), 37,9 (t,ArCH₂Ar), 14,1 (q, OCH₂CH₃).
- Spectrométrie de masse (CI) 798,1 (M⁺ calculée 798,4).
- Point de fusion 65-66°C

| - Analyse élémentaire pour | | |
|---|---|---|
| C₄₆H₅₄O₁₂ | C | H |
| calculé | 69,16 | 6,81 |
| trouvé | 68,97 | 6,96. |

### Exemple 8 : Préparation du 25,27-bis(2-hydroxyéthoxy)calix|4|arène-couronne-6 (composé 4 avec R₁ = O(CH₂CH₂O)₅, R₂ = CH₂CH₂OH et R₃ = H).

On dissout dans 50ml de tétrahydrofurane sec, 1,06mmol du 25,27-bis(éthoxycarbonylméthoxy)calix|4|arène-couronne-6 préparé dans l'exemple 7 (composé 4 avec R₁ = O(CH₂CH₂O)₅, R₂ = CH₂COOCH₂CH₃ et R₃ = H) et on ajoute à la solution 0,16g (4,24mmol) de LiAlH₄. Après deux heures d'agitation à la température ambiante, on élimine le tétrahydrofurane par distillation sous pression réduite. On traite ensuite le résidu avec 50ml d'acétate d'éthyle et 50ml de HCl à 10%, et on lave la phase organique deux fois avec de l'eau. On élimine l'acétate d'éthyle et on cristallise le résidu dans du méthanol pour obtenir le 25,27-bis(2-hydroxyéthoxy)calix|4|arène-couronne-6 (composé 4 avec R1 = O(CH₂CH₂O)₅, R₂ = CH₂CH₂OH et R₃ = H) avec un rendement de réduction de 95%.

Les caractéristiques du produit obtenu sont les suivantes :
- Point de fusion 154-155°C.
- RMN du proton (CDCl₃) :δ 7,15-7,10 (m, 8H, ArH meta), 6,95-6,90 (m, 4H, ArH para), 3,87 (s, 8H, ArCH₂Ar), 3,71 (s, 4H, O(CH₂CH₂O)₂CH₂CH₂(OCH₂CH₂)₂O), 3,67, 3,65, 3,52 et 3,40 (t, chaque 4H, O(CH₂CH₂O)₂CH₂CH₂(OCH₂CH₂)₂O), 3,60 (t, 4H, OCH₂CH₂OH, J = 6,7Hz), 3,17 (m, 4H, OCH₂CH₂OH, J = 6,7Hz), 2,38 (t, 2H, OCH₂CH₂OH).
- Spectrométrie de masse (CI) 714,0 (M⁺ calculée 714,4).

| - Analyse élémentaire pour | | |
|---|---|---|
| C₄₂H₅₀O₁₀ | C | H |
| calculé | 70,57 | 7,05 |
| trouvé | 70,39 | 7,23. |

### Exemples 9 à 14 : Extraction du césium.

Dans ces exemples, on extrait le césium présent dans une solution aqueuse ayant une concentration en acide nitrique de 1mol/l, contenant 5.10⁻⁴mol/l de CsNO₃.

Dans ce but, on met un volume de solution aqueuse en contact avec un volume de liquide organique constitué par de l'ortho-nitrophénylhexyl éther contenant 10⁻²mol/l d'extractant organique. Lorsque l'équilibre est atteint, on mesure par spectrométrie gamma, la teneur en césium du liquide organique. On détermine ensuite le pourcentage de césium extrait et le coefficient de distribution D_{Cs} qui correspond au rapport de la concentration en césium dans le liquide organique à la concentration en césium de la solution aqueuse à l'équilibre.

Dans ces exemples, on utilise comme extractant organique les ligands macrocycliques du tableau 1 dont les formules sont données sur la figure annexée. Les résultats obtenus sont également donnés dans le tableau 1.

Dans ces exemples, les exemples 9 à 12 concernent l'utilisation des calix|4|arènes de l'invention, et les exemples 13 et 14 donnés à titre comparatif illustrent l'emploi d'éthers couronnes comme extractants.

Les résultats du tableau 1 montrent que les calixarènes utilisés dans les exemples 10 et 11 (calixarènes des exemples 3 et 4) permettent d'obtenir de très bons taux d'extraction.

Par ailleurs, le césium extrait dans cet exemple peut être récupéré par mise en contact de la phase organique avec de l'eau déminéralisée.

### Exemples 15 à 19 : Extraction du strontium.

On part d'une solution aqueuse ayant une concentration en acide nitrique de lmol/l contenant 5.10⁻⁴mol/l de nitrate de strontium, et on la soumet à une extraction dans les mêmes conditions que celles des exemples 9 à 14 en utilisant une phase liquide organique constituée par de l'ortho-nitrophényl hexyl éther contenant 10⁻²mol/l d'extractant macrocyclique.

On mesure ensuite la teneur en strontium de la phase organique par spectrométrie gamma et on détermine le pourcentage de strontium extrait et le coefficient de distribution D_{Sr}.

Les extractants utilisés et les résultats obtenus sont donnés dans le tableau 2.

### Exemples 20 à 25 : Extraction du sodium.

Dans ces exemples, on part d'une solution aqueuse ayant une concentration en acide nitrique de 1mol/l contenant 5.10⁻⁴mol/l de nitrate de sodium, et on la soumet à une extraction dans les mêmes conditions que celles des exemples 9 à 14 en utilisant un extractant macrocyclique à une concentration de 10⁻²mol/l dans de l'ortho-nitrophényl hexyl éther. On mesure comme précédemment la teneur en sodium de la solution organique par spectrométrie gamma et on évalue le pourcentage de sodium extrait et le coefficient de distribution D_{Na}.

Les extractants et les résultats obtenus sont donnés dans le tableau 3.

Les résultats donnés dans les tableaux 1 à 3 montrent que les calix|4|arènes couronnes utilisés dans l'invention extraient le césium avec de bons rendements et le séparent du sodium et du strontium présents également dans les effluents issus des usines de retraitement.

A partir des résultats des tableaux 1 à 3, on peut calculer les sélectivités des différents extractants vis-à-vis de ces cations en milieu nitrique.

On trouve ainsi pour la sélectivité α(Cs/Na) = D_{Cs} / D_{Na} relative au césium par rapport au sodium les valeurs suivantes :
25,27 dimethoxy-calix|4|arène-couronne-6: 14
25,27-bis(1-propyloxy)calix|4|arène-couronne-6:10⁴
25,27-bis-(2-propyloxy)calix|4|arène-couronne-6:7.10⁴. alors que cette sélectivité est de 250 pour l'éther couronne B21C7.

Pour la sélectivité α Cs/Sr relative au césium par rapport au strontium, on trouve les valeurs suivantes :
25,27-diméthoxy-calix|4|arène-couronne-6: ≥40
25,27-bis(1-propyloxy)calix|4|arène-couronne-6: ≃ 10⁵
25,27-bis(2-propyloxy)calix|4|arène-couronne-6 ≃ 8.10⁴
alors que cette sélectivité n'est que de 7,6.10⁻² pour l'éther couronne DCH18C6.

C'est pourquoi les calixarènes de l'invention permettent d'obtenir des transports sélectifs de césium 137, à l'état de traces dans une matrice de nitrate de sodium 4M et d'acide nitrique 1M contenant d'autres produits de fission tels que du Sr 90 (simulé par ⁸⁵Sr), également à l'état de traces.

Les résultats des tableaux 1 à 3 montrent par ailleurs que les calix|4|arènes de l'invention sont de très bons extractants du césium. Ce pic de sélectivité pourrait s'expliquer par une bonne adéquation entre la taille des cavités disponibles et la taille du cation extrait.

### Exemples 26 à 30 : Extraction des actinides.

Dans ces exemples, on part d'une solution aqueuse contenant lmol/l d'acide nitrique et 4mol/l de nitrate de sodium, contenant des traces de Np 237, de Pu 239 et de Am 241, et on extrait les actinides en utilisant une phase organique liquide constituée par un extractant macrocyclique à une concentration de 10⁻²mol/l dans l'ortho-nitrophényl octyl éther ou l'orthonitrophénylhexyléther, ou un extractant connu (CMPO) à une concentration de 10⁻²mol/l dans l'ortho-nitrophényl hexyl éther.

On mesure ensuite les quantités de ²³⁷Np, ²³⁹Pu et ²⁴¹Am présentes dans la solution organique et on détermine les coefficients de distribution du neptunium, du plutonium et de l'américium entre les deux phases en contact.

Les extractants utilisés et les résultats obtenus sont donnés dans le tableau 4.

Au vu de ces résultats, on remarque que les calixl4larènes-couronnes de l'invention sont de moins bons extractants que le CMPO mais qu'ils sont plus sélectifs puisqu'ils n'extraient pas l'américium trivalent.

Ainsi, on peut effectuer avec le 25,27-diisopropoxy couronne-6-calix|4|arène des expériences de transport en disposant la phase liquide organique sous la forme d'une membrane liquide séparant la solution aqueuse de départ d'une solution de réextraction constituée par de l'eau déminéralisée, et décontaminer plus de 90% de Pu 239 et plus de 45% de Np 237 en moins de 96h, sans transporter d'américium.

Les calix|4|arènes-couronnes de l'invention peuvent aussi être utilisés en analyse, par exemple pour déterminer l'activité des différents isotopes du césium, en particulier du Cs-135. Ils peuvent être encore utilisés en détoxication.

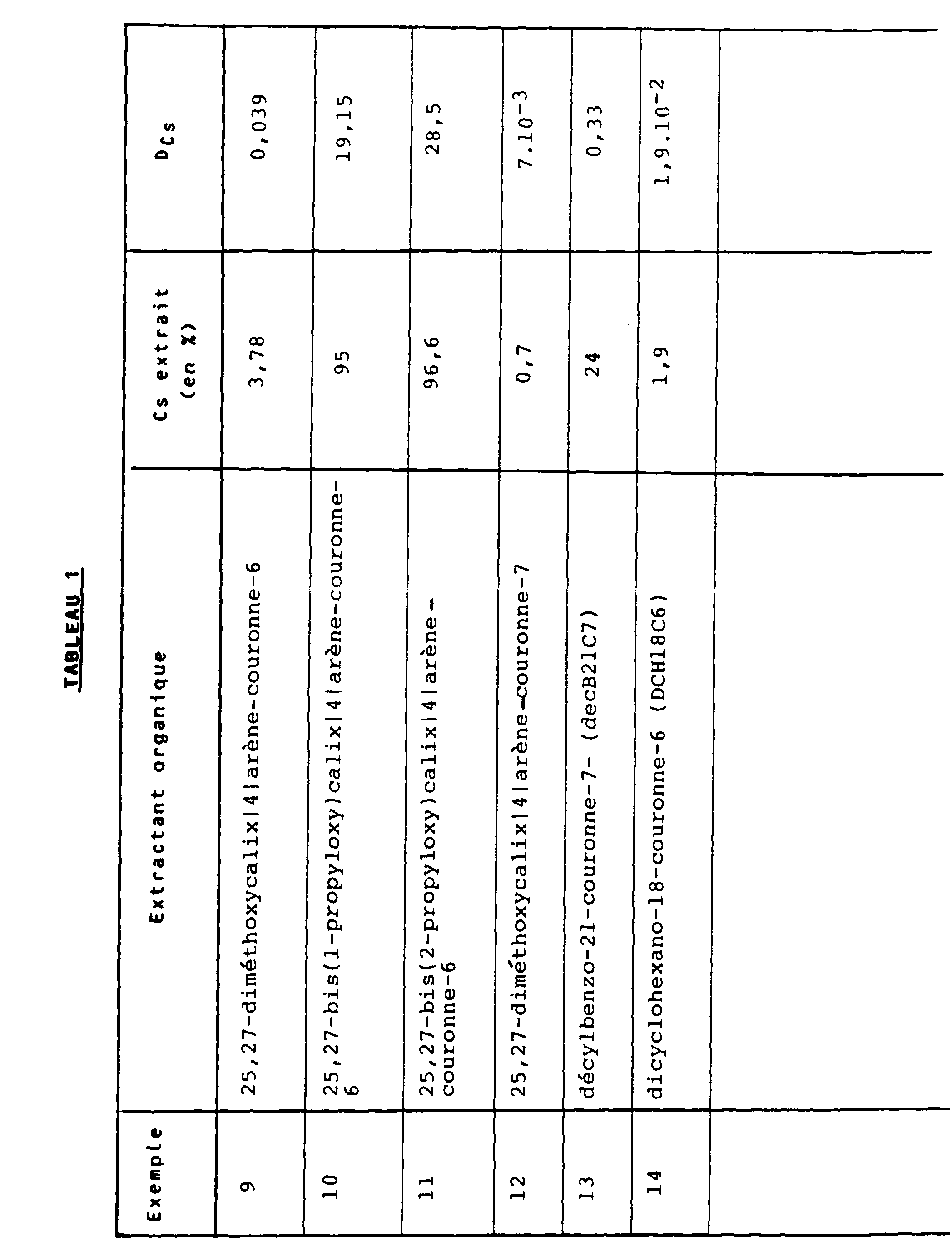

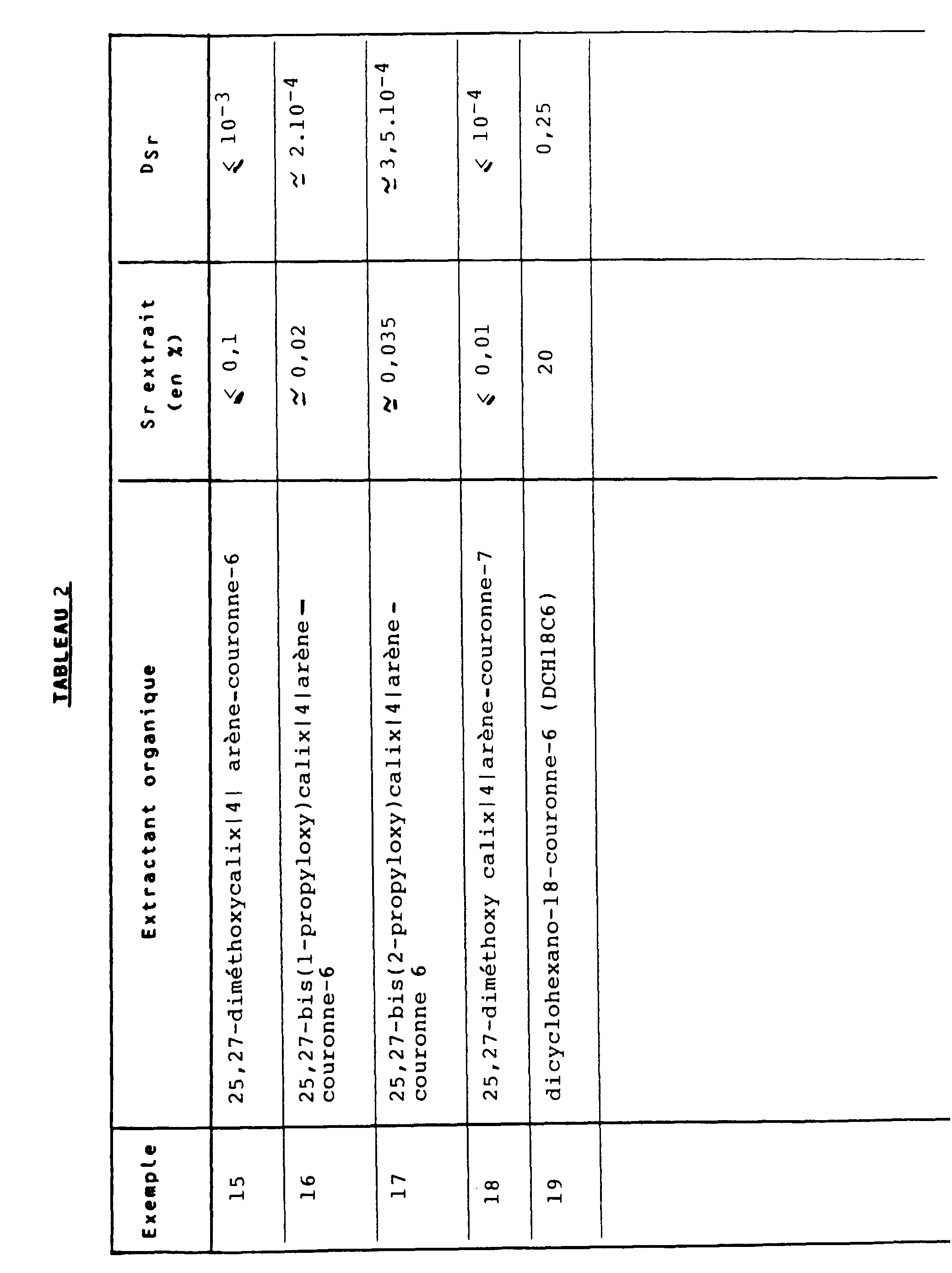

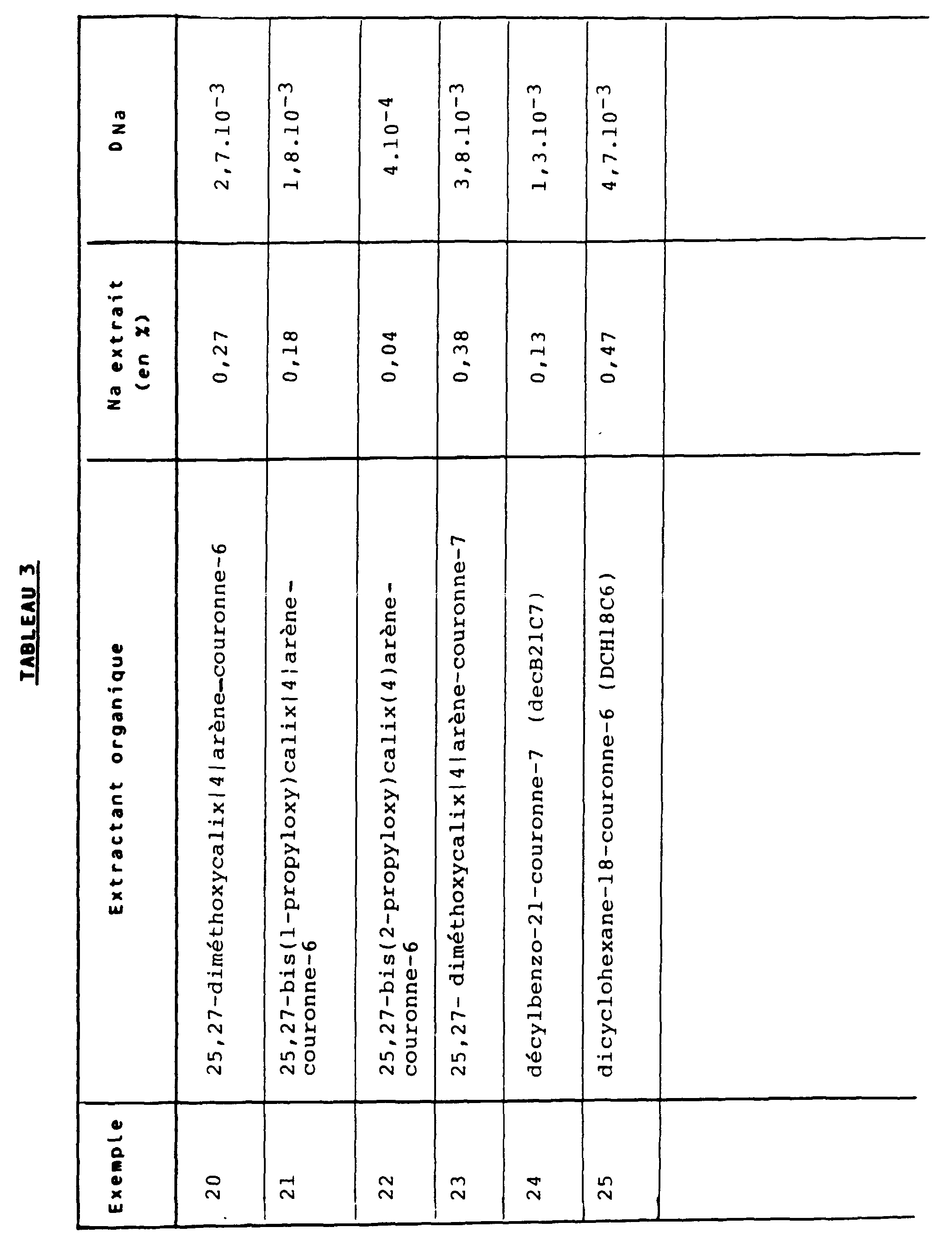

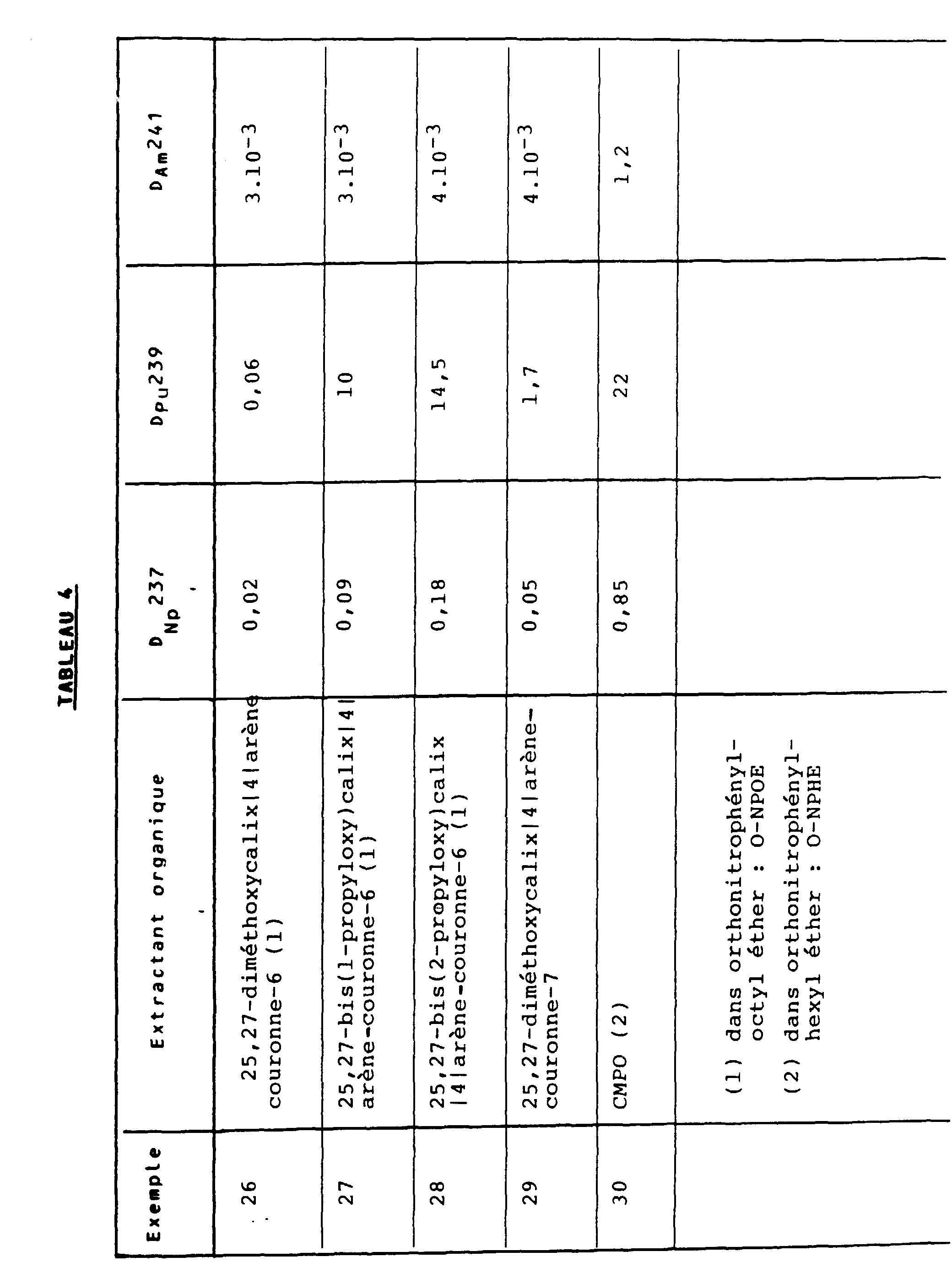

## Revendications

1. Calix|4|arène-couronne de formule : 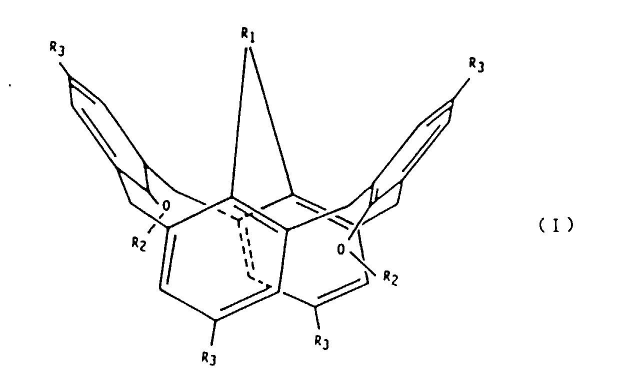 dans laquelle
- R₁ représente un groupe de formule X(CH₂CH₂X)ₘ ou de formule X(CH₂CH₂X)ₙYX(CH₂CH₂X)ₙ, dans lesquelles X représente O et/ou N(R₄) avec R₄ étant un atome d'hydrogène ou un groupe alkyle en C₁ à C₄, Y représente un groupe cycloalkylène en C₈ à C₁₄ ou arylène comportant éventuellement un hétéroatome, choisi parmi O, S et N, m est égal à 3, 4, 5 ou 6 et n est égal à 1, 2 ou 3.
- R₂ représente un atome d'hydrogène, un groupe alkyle en C₁ à C₁₂, un groupe de formule 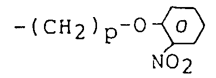 avec p étant égal à 5, 6, 7 ou 8, -CH₂-CH₂OH ou -CH₂COR₅ avec R₅ représentant un groupe de formule OR₆, NR₆R₇ ou R₆ dans lesquelles R₆ et R₇ qui peuvent être identiques ou différents, représentent un atome d'hydrogène, un groupe alkyle en C₁ à C₄ ou un groupe aryle en C₆ à C₁₄, les deux R₂ pouvant être différents, et
- R₃ représente un atome d'hydrogène ou un groupe alkyle en C₁ à C₃,
à condition que l'un ou les deux R₂ ne représentent pas un atome d'hydrogène lorsque R₁ représente X(CH₂CH₂X)m avec X = 0.

2. Calix|4|arène-couronne selon la revendication 1, caractérisé en ce que R₁ représente O(CH₂CH₂O)ₘ avec m étant égal à 5 ou 6.

3. Calixl4larène-couronne selon l'une quelconque des revendications 1 et 2, caractérisé en ce que R₃ représente un atome d'hydrogène.

4. Calix|4|arène-couronne selon l'une quelconque des revendications 1 à 3, caractérisé en ce que R₂ représente un groupe alkyle de 1 à 8 atomes de carbone, CH₂COOCH₂CH₃ ou CH₂CH₂OH.

5. Calixl4larène-couronne selon la revendication 1, caractérisé en ce que R₁ représente O(CH₂CH₂O)₅, R₂ représente -CH₂CH₂CH₃ ou -CH(CH₃)₂ et R₃ représente un atome d'hydrogène.

6. Procédé de préparation d'un calixl4larène-couronne répondant à la formule (I) de la revendication 1 avec R₂ étant un atome d'hydrogène, caractérisé en ce qu'il consiste à faire réagir un calix|4|arène de formule : 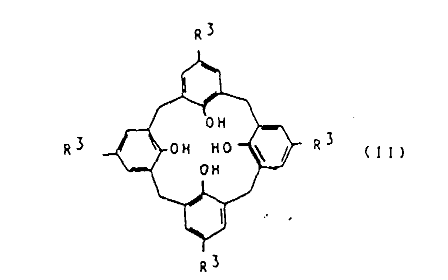 dans laquelle R₃ a la signification donnée dans la revendication 1,
- avec un composé répondant à l'une des formules suivantes :
TsX(CH₂CH₂X)ₘTs (III)
et
TsX(CH₂CH₂X)ₙYX(CH₂CH₂X)ₙTs (IV)
dans lesquelles X, m, Y et n ont les significations données dans la revendication 1, et Ts représente CH₃-C₆H₄-SO₂, et à séparer du milieu réactionnel le calixl4larène-couronne obtenu.

7. Procédé de préparation d'un calix|4|arène-couronne répondant à la formule (I) de la revendication 1 avec R₂ différent de H, caractérisé en ce qu'il comprend les étapes suivantes :
a) faire réagir un calix|4|arène de formule : 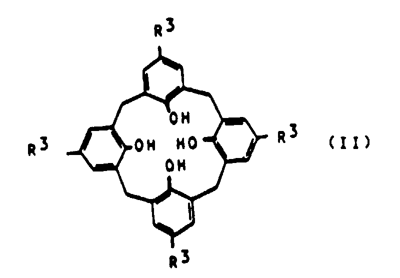 dans laquelle R₃ a la signification donnée dans la revendication 1,
- avec un composé répondant à l'une des formules suivantes :
TsX(CH₂CH₂X)ₘTs (III)
et
TsX(CH₂CH₂X)ₙYX(CH₂CH₂X)ₙTs (IV)
dans lesquelles X, m, Y et n ont la signification donnée dans la revendication 1, et Ts représente CH₃-C₆H₄-SO₂,
b) faire réagir le calix|4|arène-couronne obtenu dans l'étape a) avec un halogénure de formule R₂Z dans laquelle R₂ a la signification donnée dans la revendication 1 et Z est un atome d'halogène, et
c) séparer du milieu réactionnel le calix|4|arène-couronne de formule (I) ainsi obtenu.

8. Procédé de préparation d'un calix|4|arène-couronne répondant à la formule (I) de la revendication 1 avec R₂ différent de H, caractérisé en ce qu'il comprend les étapes suivantes :
a) faire réagir un calix|4|arène de formule : 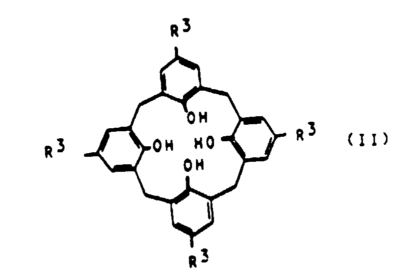 dans laquelle R₃ a la signification donnée dans la revendication 1,
avec un halogénure de formule R₂Z
dans laquelle R₂ a la signification donnée dans la revendication 1 et Z est un atome d'halogène,
b) faire réagir le calix|4|arène obtenu dans l'étape a) avec un composé répondant à l'une des formules suivantes :
TsX(CH₂CH₂X)ₘTs (III)
et
TsX(CH₂CH₂X)ₙYX(CH₂CH₂X)ₙTs (IV)
dans lesquelles X, m, Y et n ont la signification donnée dans la revendication 1, et Ts représente CH₃-C₆H₄-SO₂, et
c) séparer du milieu réactionnel le calix|4|arène-couronne obtenu.

9. Procédé pour séparer le césium d'une solution aqueuse, caractérisé en ce qu'il consiste à mettre en contact cette solution aqueuse avec une phase liquide immiscible comprenant un calix|4|arène-couronne selon l'une quelconque des revendications 1 à 5, et à récupérer ensuite le césium extrait dans cette phase liquide.

10. Procédé selon la revendication 9, caractérisé en ce que l'on récupère ensuite le césium dans une solution aqueuse de réextraction par mise en contact de la phase liquide ayant extrait le césium avec une solution aqueuse.

11. Procédé selon la revendication 10, caractérisé en ce que la solution aqueuse de réextraction est de l'eau distillée et désionisée.

12. Procédé selon l'une quelconque des revendications 10 et 11, caractérisé en ce que la phase liquide immiscible forme une membrane liquide et en ce que l'on met en contact la solution aqueuse contenant le césium avec une surface de cette membrane et en ce que l'on met en contact la solution aqueuse de réextraction avec la surface opposée de cette membrane liquide.

13. Procédé selon l'une quelconque des revendications 9 à 12, caractérisé en ce que la solution aqueuse de départ est un effluent aqueux contenant du césium, avec ou sans sodium et/ou strontium, provenant d'une installation de retraitement de combustibles irradiés.

14. Procédé pour séparer les actinides et l'américium trivalent présents dans une solution aqueuse, caractérisé en ce qu'il consiste à mettre en contact cette solution aqueuse avec une phase liquide immiscible comprenant un calix|4|arène-couronne selon l'une quelconque des revendications 1 à 5, et à récupérer ensuite les actinides extraits dans la phase liquide immiscible.

## Patentansprüche

1. Calix[4]aren-Kronenether der Formel: 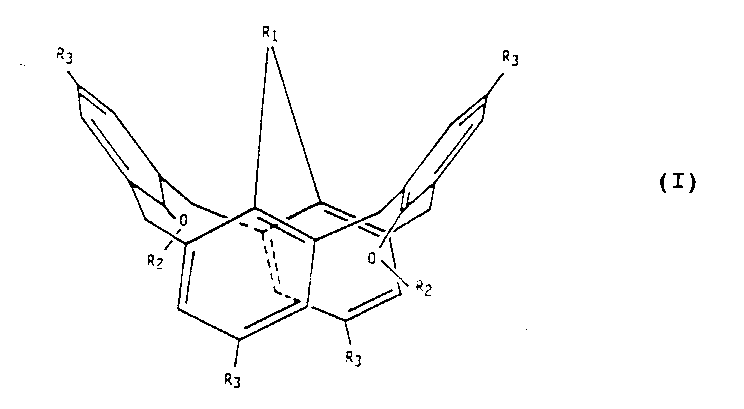 worin
- R₁ eine Gruppe der Formel X(CH₂CH₂X)ₘ oder der Formel X(CH₂CH₂X)ₙYX(CH₂CH₂CH₂X)ₙ darstellt, in denen X O und/oder N(R₄) darstellt, wobei R₄ ein Wasserstoffatom oder eine C₁-bis C₄-Alkylgruppe ist, Y eine C₅- bis C₁₄-Cycloalkylen- oder Arylengruppe darstellt, welche gegebenenfalls ein unter O, S und N gewähltes Heteroatom enthält, m gleich 3, 4, 5 oder 6 ist und n gleich 1, 2 oder 3 ist,
- R₂ ein Wasserstoffatom, eine C₁- bis C₁₂-Alkylgruppe, eine Gruppe der Formel 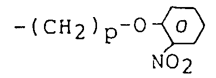 mit p gleich 5, 6, 7 oder 8, -CH₂-CH₂OH oder -CH₂COR₅ darstellt, wobei R₅ eine Gruppe der Formel OR₆, NR₆R₇ oder R₆ darstellt, in denen R₆ und R₇, die gleich oder verschieden sein können, ein Wasserstoffatom, eine C₁- bis C₄-Alkylgruppe oder eine C₆- bis C₁₄-Arylgruppe darstellen, wobei die beiden R₂ verschieden sein können, und
- R₃ ein Wasserstoffatom oder eine C₁- bis C₃-Alkylgruppe darstellt,
unter der Bedingung, daß nicht das eine oder die beiden R₂ ein Wasserstoffatom darstellen, wenn R₁ X(CH₂CH₂X)ₘ mit X = O darstellt.

2. Calix[4]aren-Kronenether nach Anspruch 1, dadurch gekennzeichnet, daß R₁ O(CH₂CH₂O)ₘ mit m gleich 5 oder 6 darstellt.

3. Calix[4]aren-Kronenether nach einem der Ansprüche 1 und 2, dadurch gekennzeichnet, daß R₃ ein Wasserstoffatom darstellt.

4. Calix[4]aren-Kronenether nach einem der Ansprüche 1 bis 3, dadurch gekennzeichnet, daß R₂ eine Alkylgruppe mit 1 bis 8 Kohlenstoffatomen, CH₂COOCH₂CH₃ oder CH₂CH₂OH darstellt.

5. Calix[4]aren-Kronenether nach Anspruch 1, dadurch gekennzeichnet, daß R₁ O(CH₂CH₂O)₅ darstellt, R₂ -CH₂CH₂CH₃ oder -CH(CH₃)₃ darstellt und R₃ ein Wasserstoffatom darstellt.

6. Verfahren zur Herstellung eines Calix[4]aren-Kronenethers entsprechend Formel (I) von Anspruch 1, wobei R₂ ein Wasserstoffatom ist, dadurch gekennzeichnet, daß es darin besteht, ein Calix[4]aren der Formel: 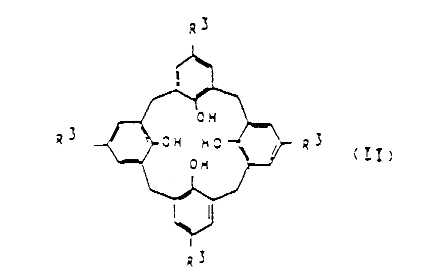 worin R₃ die in Anspruch 1 gegebene Bedeutung hat,
- mit einer Verbindung entsprechend einer der folgenden Formeln reagieren zu lassen:
TsX(CH₂CH₂X)ₘTs (III)
und
TsX(CH₂CH₂X)ₙYX(CH₂CH₂X)ₙTs (IV)
worin X, m, Y und n die in Anspruch 1 gegebene Bedeutung haben und Ts CH₃-C₆H₄-SO₂ darstellt,
und den erhaltenen Calix[4]aren-Kronenether vom Reaktionsmedium abzutrennen.

7. Verfahren zur Herstellung eines Calix[4]aren-Kronenethers entsprechend Formel (I) von Anspruch 1 mit von H verschiedenem R₂, dadurch gekennzeichnet, daß es die folgenden Schritte umfaßt:
a) ein Calix[4]aren der Formel: 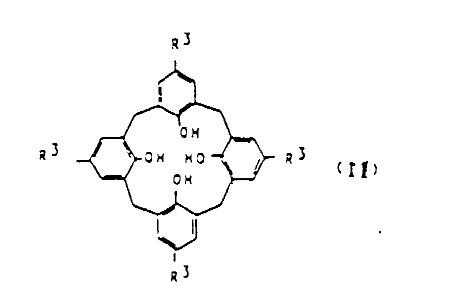 worin R₃ die in Anspruch 1 gegebene Bedeutung hat, mit einer Verbindung entsprechend einer der folgenden Formeln reagieren zu lassen:
TsX(CH₂CH₂X)ₘTs (III)
und
TsX(CH₂CH₂X)ₙYX(CH₂CH₂X)ₙTs (IV)
worin X, m, Y und n die in Anspruch 1 gegebene Bedeutung haben und Ts CH₃-C₆H₄-SO₂ darstellt,
b) den in Schritt a) erhaltenen Calix[4]aren-Kronenether mit einem Halogenid der Formel R₂Z reagieren zu lassen, worin R₂ die in Anspruch 1 gegebene Bedeutung hat und Z ein Halogenatom ist, und
c) den so erhaltenen Calix[4]aren-Kronenether der Formel (I) von dem Reaktionsmedium abzutrennen.

8. Verfahren zur Herstellung eines Calix[4]aren-Kronenethers entsprechend Formel (I) von Anspruch 1 mit von H verschiedenem R₂, dadurch gekennzeichnet, daß es die folgenden Schritte umfaßt:
a) ein Calix[4]aren der Formel: 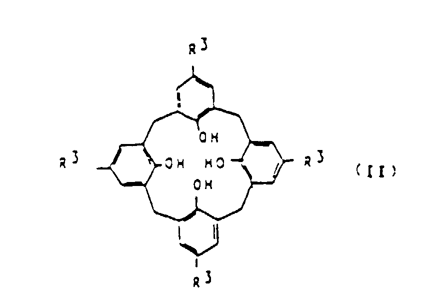 worin R₃ die in Anspruch 1 gegebene Bedeutung hat, mit einem Halogenid der Formel R₂Z reagieren zu lassen, worin R₂ die in Anspruch 1 gegebene Bedeutung hat und Z ein Halogenatom ist,
b) das in Schritt a) erhalten Calix[4]aren mit einer Verbindung entsprechend einer der folgenden Formeln reagieren zu lassen:
TsX(CH₂CH₂X)ₘTs (III)
und
TsX(CH₂CH₂X)ₙYX(CH₂CH₂X)ₙTs (IV)
worin X, m, Y und n die in Anspruch 1 gegebene Bedeutung haben und Ts CH₃-C₆H₄-SO₂ darstellt, und
c) den so erhaltenen Calix[4]aren-Kronenether von dem Reaktionsmedium abzutrennen.

9. Verfahren zur Abtrennung des Caesiums aus einer wäßrigen Lösung, dadurch gekennzeichnet, daß es darin besteht, diese wäßrige Lösung mit einer damit nicht mischbaren flüssigen Phase, welche einen Calix[4]aren-Kronenether nach einem der Ansprüche 1 bis 5 enthält, in Kontakt zu bringen und anschließend das in dieser flüssigen Phase extrahierte Caesium zu gewinnen.

10. Verfahren nach Anspruch 9, dadurch gekennzeichnet, daß man das Caesium anschließend in einer wäßrigen Reextraktionslösung dadurch gewinnt, daß man die flüssige Phase, die das Caesium extrahiert hat, mit einer wäßrigen Lösung in Kontakt bringt.

11. Verfahren nach Anspruch 10, dadurch gekennzeichnet, daß die wäßrige Reextraktionslösung destilliertes und deionisiertes Wasser ist.

12. Verfahren nach einem der Ansprüche 10 und 11, dadurch gekennzeichnet, daß die nicht mischbare flüssige Phase eine flüssige Membran bildet, daß man die das Caesium enthaltende wäßrige Lösung mit einer Oberfläche dieser Membran in Kontakt bringt und daß man die wäßrige Reextraktionslösung mit der entgegengesetzten Oberfläche dieser flüssigen Membran in Kontakt bringt.

13. Verfahren nach einem der Ansprüche 9 bis 12, dadurch gekennzeichnet, daß die wäßrige Ausgangslösung ein Caesium zusammen mit oder ohne Natrium und/oder Strontium enthaltendes Abwasser ist, welches einer Wiederaufarbeitungsanlage für bestrahlte Kernbrennstoffe entstammt.

14. Verfahren zur Abtrennung der Actiniden und des dreiwertigen Americiums, die in einer wäßrigen Lösung vorhanden sind, dadurch gekennzeichnet, daß es darin besteht, diese wäßrige Lösung mit einer damit nicht mischbaren flüssigen Phase, welche einen Calix[4]aren-Kronenether nach einem der Ansprüche 1 bis 5 enthält, in Kontakt zu bringen und anschließend die in der nicht mischbaren flüssigen Phase extrahierten Actiniden zu gewinnen.

## Claims

1. Crown calix|4|arene of formula: 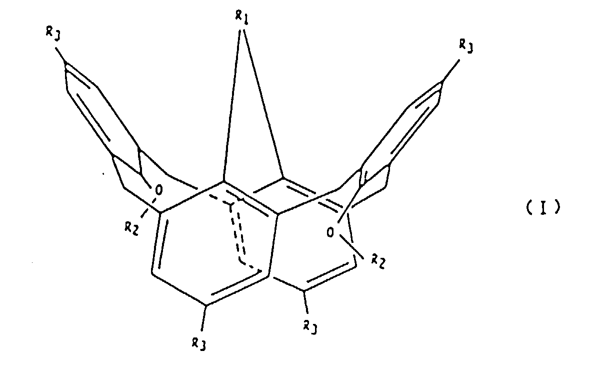 in which
- R₁ represents a group of formula X(CH₂CH₂X)ₘ or formula X(CH₂CH₂X)ₙ YX(CH₂CH₂X)ₙ in which X represents O and/or N(R₄) with R₄ representing a hydrogen atom or a C₁ to C₄ alkyl group, Y a C₆ to C₁₄ cycloalkylene or arylene group optionally having a heteroatom, me being equal to 3, 4, 5 or 6 and n equal to 1, 2 or 3,
- R₂ represents a hydrogen atom, a C₁ to C₁₂ alkyl group, a group of formula 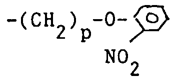 with p being equal to 5, 6, 7 or 8, -CH₂-CH₂OH or -CH₂COR₅ with R₅ representing a group of formula OR₆, NR₆R₇ or R₆, in which R₆ and R₇, which can be the same or different, represent a hydrogen atom, a C₁ to C₄ alkyl group or a C₆ to C₁₄ aryl group, the two R₂ possibly being different and
- R₃ represents a hydrogen atom or a C₁ to C₃ alkyl group, provided that one or both R₂ do not represent a hydrogen atom when R₁ represents X(CH₂CH₂X)ₘ with X = O.

2. Crown calix|4|arene according to claim 1, characterized in that R₁ represents O(CH₂CH₂O)ₘ with m being equal to 5 or 6.

3. Crown calix|4|arene according to either of the claims 1 and 2, characterized in that R₃ represents a hydrogen atom.

4. Crown calix|4|arene according to any one of the claims 1 to 3, characterized in that R₂ represents an alkyl group with 1 to 8 carbon atoms, CH₂COOCH₃ or CH₂CH₂OH.

5. Crown calix|4|arene according to claim 1, characterized in that R₁ represents O(CH₂CH₂O)₅, R₂ represents -CH₂CH₂CH₃ or -CH(CH₃)₂ and R₃ represents a hydrogen atom.

6. Process for the preparation of a crown calix|4|arene according to formula (I) of claim 1 with R₂ being a hydrogen atom, characterized in that it consists of reacting a calix|4|arene of formula: 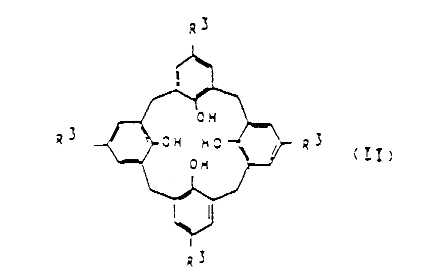 in which R₃ has the meaning given in claim 1 with a compound complying with one of the following formulas:
TsX(CH₂CH₂X)ₘTs (III)
and
TsX(CH₂CH₂X)ₙYX(CH₂CH₂X)ₙTs (IV)
in which X, M, Y and n have the meanings given in claim 1 and Ts represents CH₃-C₆H₄SO₂ and separating the crown calix|4|arene obtained from the reaction medium.

7. Process for the preparation of a crown calix|4|arene according to formula (I) of claim 1 with R₂ being different from H, characterized in that it comprises the following stages:
a) reacting the calix|4|arene of formula: 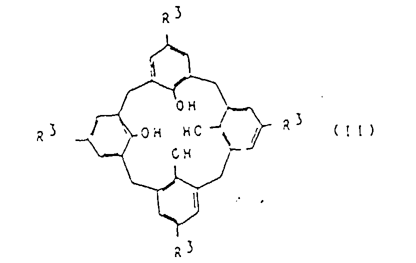 in which R₃ has the meaning given in claim 1 with a compound complying with one of the following formulas:
TsX(CH₂CH₂X)ₘTs (III)
and
TsX(CH₂CH₂X)ₙYX(CH₂CH₂X)ₙTs (IV)
in which X, m, Y and n have the meanings given in claim 1 and Ts represents CH₃-C₆H₄-SO₂,
b) reacting the crown calix|4|arene obtained in stage a) with a halide of formula R₂Z, in which R₂ has the meaning given in claim 1 and Z is a halogen atom and
c) separating the crown calix|4|arene of formula (I) obtained in this way from the reaction medium.

8. Process for the preparation of a crown calix|4|arene according to formula (I) of claim 1 with R₂ different from H, characterized in that it comprises the following stages:
a) reacting a calix|4|arene of formula: 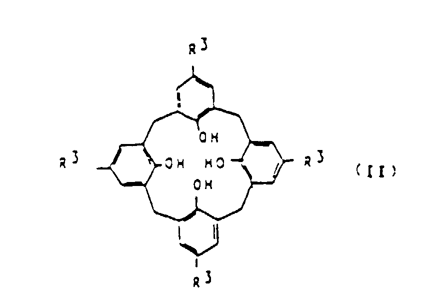 in which R₃ has the meaning given in claim 1, with a halide of formula R₂Z, in which R₂ has the meaning given in claim 1 and Z is a halogen atom,
b) reacting the calix|4|arene obtained in stage a) with a compound complying with one of the following formulas:
TsX(CH₂CH₂X)ₘ Ts (III)
and
TsX(CH₂CH₂X)ₙYX(CH₂CH₂X)ₙTs (IV)
in which X, m, Y and n have the meanings given in claim 1 and Ts represents CH₃-C₆H₄-SO₂ and
c) separating the crown calix|4|acene obtained from the reaction medium.

9. Process for separating cesium from an aqueous solution, characterized in that it consists of contacting said aqueous solution with an immiscible liquid phase incorporating a crown calix|4|arene according to any one of the claims 1 to 5 and then recovering the cesium extracted in said liquid phase.

10. Process according to claim 9, characterized in that the cesium is then extracted in an aqueous reextraction solution by contacting the liquid phase which has extracted the cesium with an aqueous solution.

11. Process according to claim 10, characterized in that the aqueous reextraction solution is distilled and deionized water.

12. Process according to either of the claims 10 and 11, characterized in that the immiscible liquid phase forms a liquid membrane and in that the cesium-containing aqueous solution is contacted with one surface of said membrane and the aqueous reextraction solution is contacted with the opposite surface of said liquid membrane.

13. Process according to any one of the claims 9 to 12, characterized in that the aqueous starting solution is an aqueous, cesium-containing effluent, with or without sodium and/or strontium obtained from an irradiated fuel reprocessing installation.

14. Process for separating the actinides and trivalent americium present in an aqueous solution, characterized in that it consists of contacting said aqueous solution with an immiscible liquid phase incorporating a crown calix|4|arene according to any one of the claims 1 to 5 and then recovering the extracted actinides in the immiscible liquid phase.
